Europäisches Patentamt

European Patent Office

Office européen des brevets

(19)

(11) Veröffentlichungsnummer : **0 044 438**
**B2**

(12) **NEUE EUROPÄISCHE PATENTSCHRIFT**

(45) Veröffentlichungstag der neuen Patentschrift :
21.01.87

(51) Int. Cl.⁴ : **C 07 D249/08**

(21) Anmeldenummer : 81104977.4

(22) Anmeldetag : 26.06.81

(54) Verfahren zur Herstellung von 1,2,4-Triazol.

(30) Priorität : 07.07.80 AT 3533/80

(43) Veröffentlichungstag der Anmeldung :
27.01.82 Patentblatt 82/04

(45) Bekanntmachung des Hinweises auf die Patenterteilung : 16.11.83 Patentblatt 83/46

(45) Bekanntmachung des Hinweises auf die Entscheidung
über den Einspruch : 21.01.87 Patentblatt 87/04

(84) Benannte Vertragsstaaten :
BE CH DE FR GB IT LI NL SE

(56) Entgegenhaltungen :
EP-A- 0 003 500
EP-A- 0 030 209
DE-A- 2 943 265
LU-A- 61 617
JOURNAL OF THE AMERICAN CHEMICAL SOCIETY,
Band 77, 10. Februar 1955, Seiten 621-624 U.S.A. C.
AINSWORTH et al.: "Isomeric and nuclear-substituted beta-aminoethyl-1,2,4-triazoles"

(73) Patentinhaber : CHEMIE LINZ AKTIENGESELLS-
CHAFT
St. Peter-Strasse 25
A-4020 Linz (AT)
BE CH FR GB IT LI NL SE
Lentia Gesellschaft mit beschränkter Haftung
Arabellastrasse 4 Postfach 81 05 08
D-8000 München 81 (DE)
DE

(72) Erfinder : Beer, Hans, Dr.
Wallrissstrasse 26
A-1180 Wien (AT)

## Beschreibung

Die vorliegende Erfindung betrifft ein Verfahren zur Herstellung von reinem 1,2,4-Triazol in sehr guter Ausbeute durch Umsetzung von Hydrazin mit Formamid bei erhöhter Temperatur bei einem Molverhältnis von Formamid zu Hydrazin, das 2 : 1 beträgt oder darüber liegt.

1,2,4-Triazolderivate haben große Bedeutung als Fungizide, Heilmittel und dergleichen erlangt. Der Bedeutung entsprechend sind zahlreiche Arbeiten erschienen, die sich mit der Herstellung derselben befassen (siehe die Zusammenfassung in « Chemical Reviews » 1961, Seite 88 ff.).

Für die Herstellung des Grundstoffes 1,2,4-Triazol sind schon frühzeitig Formamid und Hydrazinhydrat als Rohstoffe verwendet worden. Trotz Steigerung der Temperatur bis auf 280 °C konnten, wenn ohne Druckanwendung gearbeitet wird, nur Ausbeuten von etwa 30 % der Theorie erreicht werden. Unter Anwendung von Druck, einer Temperatur von 200 °C und unter Einsatz von überschüssigem Ammoniak gelang es, die Ausbeute auf 70 bis 80 % zu steigern, wobei die Reaktionszeit 24 Stunden betrug (J. Am. Chem. Soc. Vol. 77, 1955, 621-624).

Aus der Europäischen Patentanmeldung 3500 ist ein Verfahren zur Herstellung von 1,2,4-Triazol durch Umsetzung von Hydrazin mit Formamid bekannt, bei welchem im Temperaturbereich von 100 bis 250 °C in Gegenwart von Ammoniak Hydrazin mit Formamid im Molverhältnis von 1 : 2,0 bis 1 : 2,7 umgesetzt wird, wobei in mehreren Reaktionsstufen mit steigender Reaktionstemperatur gearbeitet wird. Der in den einzelnen Stufen gebildete Ammoniak wird dabei in die vorhergehenden Stufen zurückgeführt. Dieses Verfahren liefert Ausbeuten über 90 % der Theorie, ist aber, da es eine Reaktionskaskade benötigt, sehraufwendig.

Aus der LU-PS 61 617 ist bekannt 1,2,4-Triazol durch Erhitzen von 1 Mol Hydrazin-Hydrat mit etwa 3 Mol Formamid herzustellen. Um gute Ausbeuten zu erzielen ist es erforderlich in mehreren Stufen zu arbeiten.

Es wurde nun überraschenderweise gefunden, daß mit Leichtigkeit und Sicherheit reines 1,2,4-Triazol in ausgezeichneten Ausbeuten in einstufiger Reaktion und ohne Einleitung von Ammoniak erzeugt werden kann, wenn man die Reaktionspartner Formamid und Hydrazin in einem bestimmten Molverhältnis anwendet, dafür sorgt, daß für die Umsetzung mit dem Hydrazin stets überschüssiges, vorerhitztes Formamid zur Verfügung steht und die Einwirkung der beiden Reaktionskomponenten bei einer nicht unter 160 °C liegenden Temperatur erfolgt. Auf diese Weise erzielt man kürzere Reaktionszeiten ; ferner bleibt der Anteil an entstehenden Nebenprodukten sehr gering.

Das erfindungsgemäße Verfahren zur Herstellung von 1,2,4-Triazol durch Umsetzung von Hydrazin mit Formamid ist demnach dadurch gekennzeichnet, daß in einer einzigen Reaktionsstufe mit einem Molverhältnis von Formamid zu Hydrazin von 2 : 1 bis 2,2 : 1 gearbeitet wird, wobei das Hydrazin als solches oder in Form seines Hydrats in überschüssiges, auf eine Temperatur im Bereich von 160 °C bis 200 °C vorerhitztes Formamid langsam eindosiert und während des Eindosierens die Temperatur in diesem Bereich gehalten wird, worauf anschließend bei Temperaturen von 160-240 °C die Reaktion vollendet wird.

Für das Gelingen der erfindungsgemäßen Umsetzung ist es wesentlich, daß das Formamid stets gegenüber dem Hydrazin im Überschuß vorliegt. Es muß daher während des Eintragens des Hydrazins, das bevorzugt als hydrazinhydrat eingesetzt wird, für eine rechtzeitige Ergänzung des Formamids gesorgt werden, wobei die Temperatur von 160 °C nicht unterschritten werden darf. Bevorzugt wird gleich die ganze umzusetzende Menge an Formamid vorerhitzt und dann erst das Hydrazin bzw. Hydrazinhydrat eingetragen.

Günstigerweise soll die Temperatur während der Eintragung und bei der anschließenden Reaktion zwischen 165-190 °C gehalten werden, wobei ein Temperaturbereich vvn 175-185 °C besonders bevorzugt ist.

Bei der praktischen Durchführung des erfindungsgemäßen Verfahrens hat es sich als zweckmäßig erwiesen, so vorzugehen, daß man das Hydrazin von einem Hochgefäß aus oder mittels einer Pumpe in das vorgelegte und vorerhitzte Formamid einträgt, vorzugsweise eindüst, wodurch sofort eine gute Verteilung entsteht. Vorzugsweise wird dabei das Hydrazin in Bodennähe des im Reaktor erhitzt vorliegenden Formamids unter geringem, zur Überwindung des auf der Eintrittsstelle des Hydrazins lastenden hydrostatischen Druckes ausreichenden Überdruck eindosiert und dort mit dem Formamid umgesetzt. Als Überdruck über dem hydrostatischen Druck genügt bei der Anwendung eines Einführungsrohres ein Überdruck von etwa 0,1 bar. Bei Verwendung von Düsen für die Einführung des Hydrazins sind in Abhängigkeit von der Düsenkonstruktion im allgemeinen Überdrücke von 1-3 bar günstig. Günstig ist es auch, während der Umsetzung zu rühren. Die Reaktion setzt schnell ein, es entwickeln sich Wasser- und Ammoniakdämpfe, die die Reaktionsmasse durchstreichen. Das Reaktionsgefäß trägt vorteilhafterweise eine Füllkörperkolonne, die die Funktion hat, mitgerissenes Formamid zu kondensieren und Wasserdampf und Ammoniakdampf abdestillieren zu lassen. Das theoretische Molverhältnis, nämlich 2 Molanteile Formamid und 1 Molanteil Hydrazin, muß umsoweniger zugunsten des Formamids überschritten werden, je wirksamer diese Kolonne arbeitet.

Nachdem das gesamte, für die Umsetzung erforderliche Hydrazin eingeführt, insbesondere eingedüst ist, wird das reaktionsgemisch zweckmäßig noch eine halbe Stunde lang bei der Reaktionstemperatur gerührt, wobei die Temperatur gegen Ende der Reaktion gegenüber der Eintragungstemperatur etwas gesteigert werden kann. Nach Beendigung der Reaktion wird zweckmäßig auf etwa 140 °C und anschließend auf 130 °C abgekühlt. Da das als Reaktionsprodukt erhaltene 1,2,4-Triazol auf Grund seiner Reinheit sonst kristallisieren würde, empfiehlt es sich, es noch im flüssigen Zustand bei etwa 130 °C aus dem Reaktor zu entleeren.

Beim erfindungsgemäßen Verfahren werden nahezu theoretische Ausbeuten erzielt. Das rohe, schwach gelb gefärbte Triazol kann in einem geeigneten Lösungsmittel, z. B. Isopropylalkohol, in ein gut kristallisierendes weißes Produkt, das einen scharfen Schmelzpunkt zeigt, umkristallisiert werden.

Beispiel

Es wird ein Reaktor verwendet, der aus einem Edelstahlkessel mit einem Fassungsvermögen von etwa 200 l besteht. Der Reaktor ist mit einer 1 m langen Kolonne, Rührer, Thermometer und bis knapp an den Boden des Gefäßes reichendem Einspritzrohr ausgestattet. Durch das Einspritzrohr wird während der Reaktion Hydrazinhydrat (ca. 64 %iges Hydrazin) eingeführt. Es werden 60 kg Formamid vorgelegt und auf 180. °C Innentemperatur erhitzt. Nun werden unter Rühren im Verlaufe von etwa 4 Stunden 30 kg Hydrazinhydrat unter schwachem Überdruck so eindosiert, daß kein Hydrazin mitgerissen wird und die Temperatur zwischen 175 und 180 °C gehalten werden kann. Die Geschwindigkeit der Hydrazinzugabe ist von der Wirksamkeit der Hydrazinzugabe ist von der Wirksamkeit der Kolonne und des absteigenden Kühlers abhängig. Nach der Zugabe des Hydrazins wird noch 30 Minuten bei 180 bis 180 °C gerührt und dann auf etwa 140 °C abgekühlt. Der noch flüssige Inhalt des Reaktors wird bei etwa 130 bis 140 °C ausgepreßt und nach dem Erkalten mit entsprechender Vorrichtung zerkleinert. Man erhält so 1,2,4-Triazol, das nur schwach gelb gefärbt ist und geringe Mengen Formamid enthält. Die Ausbeute beträgt etwa 45 kg an 93 %igem Produkt.

Patentansprüche

1. Verfahren zur Herstellung von 1,2,4-Triazol durch Umsetzung von Hydrazin und Formamid bei erhöhter Temperatur und einem Molverhältnis von Formamid zu Hydrazin von mindestens 2 : 1, dadurch gekennzeichnet, daß in einer einzigen Reaktionsstufe mit einem Molverhältnis von Formamid zu Hydrazin von 2 : 1 bis 2,2 : 1 gearbeitet wird, wobei das Hydrazin als solches oder in Form seines Hydrats in überschüssiges, auf eine Temperatur im Bereich von 160 °C bis 200 °C vorerhitztes Formamid langsam eindosiert und während des Eindosierens die Temperatur in diesem Bereich gehalten wird, worauf anschließend bei Temperaturen von 160-240 °C die Reaktion vollendet wird.

2. Verfahren nach Anspruch 1, dadurch gekennzeichnet, daß die gesamte Menge Formamid vorgelegt wird, bevor mit der Eintragung des Hydrazins begonnen wird.

3. Verfahren nach Anspruch 1, dadurch gekennzeichnet, daß ein Teil des Formamids vorgelegt und während des Eindosierens des Hydrazins das Formamid in der Weise ergänzt wird, daß es gegenüber dem Hydrazin stets im Überschuß bleibt.

4. Verfahren nach den Ansprüchen 1 bis 3, dadurch gekennzeichnet, daß die Temperatur während des Eindosierens und der Reaktion auf 165-190 °C gehalten wird.

5. Verfahren nach Anspruch 4, dadurch gekennzeichnet, daß die Temperatur auf 175-185 °C gehalten wird.

6. Verfahren nach den Ansprüchen 1-5, dadurch gekennzeichnet, daß man das Hydrazin in Bodennähe des im Reaktionsgefäß vorliegenden, auf Reaktionstemperatur erhitzten Formamids unter einem, zur Überwindung des auf der Eintrittsstelle des Hydrazins lastenden hydrostatischen Druckes ausreichenden Überdruck eindosiert.

7. Verfahren nach Anspruch 6, dadurch gekennzeichnet, daß das Hydrazin eined¸st wird.

8. Verfahren nach den Ansprüchen 1 bis 7, dadurch gekennzeichnet, daß während der Reaktion durch gebildeten Wasserdampf und Ammoniak mitgerissenes Formamid kondensiert und in das Reaktionsgemisch zurückgeführt wird.

9. Verfahren nach den Ansprüchen 1 bis 8, dadurch gekennzeichnet, daß das Reaktionsgemisch während der Umsetzung gerührt wird.

**Claims**

1. A process for the preparation of 1,2,4-triazole by reacting hydrazine and formamide, at elevated temperature and in a molar ratio of formamide to hydrazine of at least 2 : 1, comprising operating in a signle reaction stage with a molar ratio of formamide to hydrazine of 2 : 1 to 2,2 : 1, hydrazine as such or in the form of its hydrate being slowly metered into excess formamide preheated to a temperature in the range of 160 °C to 200 °C and the temperature being maintained within this range during metering-in, whereupon the reaction is subsequently completed at temperatures of 160-240 °C.

2. A process as claimed in claim 1, wherein the total quantity of formamide is introduced initially before the introduction of hydrazine commences.

3. A process as claimed in claim 1, wherein part of the formamide is introduced initially and, during the metering-in of the hydrazine, the formamide is added in such a way that it is always in excess over the hydrazine.

4. A process as claimed in claims 1 to 3, where in the temperature is maintained at 165-190 °C during the metering-in and the reaction.

5. A process as claimed in claim 4, wherein the temperature is maintained at 175-185 °C.

6. A process as claimed in claims 1 to 5, wherein the hydrazine is metered-in, near to the bottom of the formamide heated to the reaction temperature and present in the reaction vessel, under an elevated pressure which is sufficient to overcome the hydrostatic pressure applying at the entry point of the hydrazine.

7. A process as claimed in claim 6, wherein the hydrazine is injected through a jet.

8. A process as claimed in claims 1 to 7, wherein the formamide entrained during the reaction by the water vapor and ammonia formed is condensed and is returned into the reaction mixture.

9. A process as claimed in claims 1 to 8, wherein the reaction mixture is stirred during the reaction.

**Revendications**

1. Procédé pour la production de 1,2,4-triazole par réaction d'hydrazine et de formamide à température élevée et selon un rapport molaire entre le formamide et l'hydrazine d'au moins 2 : 1, caractérisé en ce qu'on opère en un seul stade de réaction avec un rapport molaire entre le formamide et l'hydrazine allant de 2 : 1 à 2,2 : 1, l'hydrazine étant introduite lentement de façon dosée, telle quelle ou sous forme de son hydrate, dans du formamide en excès préchauffé à une température comprise dans une gamme allant de 160 °C à 200 °C, la température étant maintenue dans cette gamme pendant l'introduction dosée, la réaction étant ensuite complétée à des températures allant de 160 à 240 °C.

2. Procédé suivant la revendication 1, caractérisé en ce que la quantité totale de formamide est présente au début de la réaction, avant que l'introduction de l'hydrazine ne commence.

3. Procédé suivant la revendication 1, caractérisé en ce qu'une partie de formamide est présente avant la réaction, le formamide étant complété pendant l'introduction dosée de l'hydrazine de telle sorte qu'il demeure toujours en excès par rapport à l'hydrazine.

4. Procédé suivant les revendications 1 à 3, caractérisé en ce que la température est maintenue pendant l'introduction dosée et pendant la réaction entre 165 et 190 °C.

5. Procédé suivant la revendication 4, caractérisé en ce que la température est maintenue à 175-185 °C.

6. Procédé suivant les revendications 1 à 5, caractérisé en ce qu'on introduit de façon dosée l'hydrazine au voisinage du fond du formamide présent dans le récipient de réaction et chauffé à la température de réaction, sous une surpression suffisante pour surmonter la pression hydrostatique s'exerçant au point d'introduction de l'hydrazine.

7. Procédé suivant la revendication 6, caractérisé en ce que l'hydrazine est introduite au moyen d'une ou plusieurs buses.

8. Procédé suivant les revendications 1 à 7, caractérisé en ce que le formamide entraîné pendant la réaction par la vapeur d'eau et l'ammoniac formés est condensé et renvoyé au mélange réactionnel.

9. Procédé suivant les revendications 1 à 8, caractérisé en ce qu'on agite le mélange réactionnel pendant la réaction.